(19) Europäisches Patentamt
European Patent Office
Office européen des brevets

(11) Publication number: **0 123 830 B1**

(12) **EUROPEAN PATENT SPECIFICATION**

(45) Date of publication of patent specification: **26.10.88**

(51) Int. Cl.$^4$: **C 07 C 120/00,** C 07 C 121/43

(21) Application number: **84102318.7**

(22) Date of filing: **05.03.84**

# (54) Novel process for the preparation of aminonitriles useful for the preparation of herbicides.

(30) Priority: **28.03.83 US 479301**

(43) Date of publication of application:
**07.11.84 Bulletin 84/45**

(45) Publication of the grant of the patent:
**26.10.88 Bulletin 88/43**

(84) Designated Contracting States:
**BE CH DE FR GB IT LI NL SE**

(56) References cited:
**DE-A- 669 808**
**GB-A-1 566 523**
**US-A-3 620 710**

**J. MARCH: "Advanced Organic Chemistry - Reactions, Mechanisms, and Structure", 2nd edition, 1977, pages 874-875, McGraw-Hill International Book Co., Auckland, US;**

**The file contains technical information submitted after the application was filed and not included in this specification**

(73) Proprietor: **AMERICAN CYANAMID COMPANY**
**1937 West Main Street P.O. Box 60**
**Stamford Connecticut 06904-0060 (US)**

(72) Inventor: **Stepek, Walter Joseph**
**126 Estates Boulevard Apt. 161**
**Trenton New Jersey 08610 (US)**
Inventor: **Nigro, Matthew Michael**
**12 Heritage Way**
**Lawrenceville New Jersey 08648 (US)**

(74) Representative: **Wächtershäuser, Günter, Dr.**
**Tal 29**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 123 830 B1**

Courier Press, Leamington Spa, England.

**Description**

The invention is a process for the preparation of aminonitriles of formula (I)

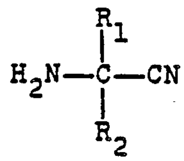

(I)

wherein $R_1$ is $C_1$—$C_4$ alkyl; $R_2$ is $C_1$—$C_4$ alkyl or $C_3$—$C_6$ cycloalkyl, and when $R_1$ and $R_2$ are not the same, the racemic mixtures and the optical isomers thereof.

The most preferred formula (I) compound is

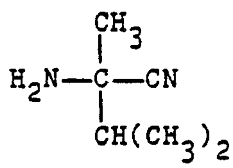

The above formula (I) aminonitriles are useful and valuable intermediates by themselves, and especially when first hydrolyzed to the corresponding amides of formula (II):

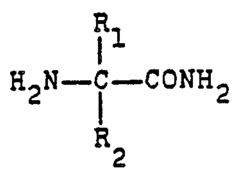

(II)

wherein $R_1$ and $R_2$ are as hereinabove defined, for the preparation of herbicidal 2-(5,5-disubstituted-4-oxo-2-imidazolin-2-yl)nicotinic acids and 3-quinoline-carboxylic acids of formula (III):

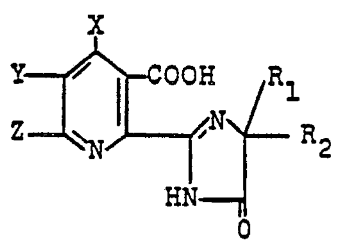

(III)

wherein $R_1$ and $R_2$ are as defined above; X is hydrogen or $C_1$—$C_4$ alkyl, Y is selected from hydrogen, halogen, $C_1$—$C_4$ alkyl, $C_1$—$C_4$ alkoxy, trifluoromethyl, trichloromethyl, difluoromethoxy, diloweralkylamino, $C_1$—$C_4$ alkylthio, phenyl, phenoxys or phenyl or phenoxy substituted with one $C_1$—$C_4$ alkyl, $C_1$—$C_4$ alkoxy or halogen; Z is selected from hydrogen, $C_1$—$C_4$ alkyl, trifluoromethyl, trichloromethyl, phenyl or phenyl substituted with one $C_1$—$C_4$ alkyl, $C_1$—$C_4$ alkoxy or halogen; and, when taken together Y and Z may form a ring in which YZ is represented by the structure: $-(CH_2)n$-wherein n is an integer selected from 3 to 5 provided that X is hydrogen; or YZ is

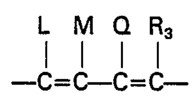

where L, M, Q and $R_3$ reach represent members selected from hydrogen, halogen, $C_1$—$C_4$ alkyl, $C_1$—$C_4$ alkoxy, $C_1$—$C_4$ haloalkyl, difluoromethoxy, diloweralkylamino, $C_1$—$C_4$ alkylthio, nitro, phenyl, phenoxy, or mono-substituted phenyl or phenoxy where the substituent is one $C_1$—$C_4$ alkyl, $C_1$—$C_4$ alkoxy or halogen, with the proviso that only one of the L, M, Q or $R_3$, may represent a substituent other than hydrogen, halogen, $C_1$—$C_4$ alkyl or $C_1$—$C_4$ alkoxy; the racemates, the optical isomers thereof, and the salts thereof.

Additionally, said aminonitriles are also useful and valuable intermediates for the preparation of herbicidal 2 and 6-(5,5-disubstituted-4-oxo-2-imidazolin-2-yl)benzoic acids and esters of formula (III):

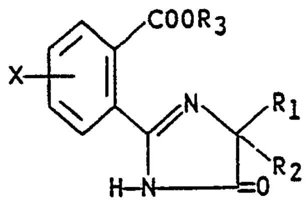

(IIIA)

wherein X is hydrogen, alkyl $C_1$—$C_3$, halogen or nitro; $R_1$ is akyl $C_1$—$C_4$; $R_2$ is alkyl $C_1$—$C_6$, cycloalkyl $C_3$—$C_6$, alkenyl $C_2$—$C_4$, phenyl halophenyl or benzyl or when $R_1$ and $R_2$ are taken together with the carbon to which they are attached they may represent cycloalkyl $C_3$—$C_6$ optionally substituted with methyl; $R_3$ is hydrogen, alkyl $C_1$—$C_{12}$ optionally substituted with one $C_1$—$C_3$ alkoxy group or one $C_3$—$C_6$ cycloalkyl group or one phenyl group or one furyl group or with one to three halogen substituent(s) preferably chlorine, alkenyl $C_3$—$C_5$ optionally substituted with one or two $C_1$—$C_3$ alkyl group(s) or one phenyl group or with one to two halogen substituent(s) preferably chlorine, alkynyl $C_3$—$C_5$ optionally substituted with one or two $C_1$—$C_3$ alkyl group(s) or one phenyl group or with one to two halogen substituent(s) preferably chlorine, benzyl, cyclohexenyl-methyl, ethynylcyclohexyl, ethynylalkyl, pentadienyl or cycloalkyl $C_3$—$C_6$ optionally substituted with one or two $C_1$—$C_3$ alkyl group(s); or a salt-forming cation or alkali metals, ammonium and aliphatic ammonium; and when $R_1$ and $R_2$ are not the same the optical isomers and the isomeric mixtures thereof; and except when $R_3$ is a salt-forming cation, the acid addition salts thereof.

The above formula (III) herbicides are described in the United States Patent of Marinus Los No. 4,368,068 which issued on January 20, 1987.

The above formula (IIIA) herbicides are described in the United States Letters Patent of Marinus Los, No. 4,188,487, which issued on February 12, 1980.

We find, that by the novel process of the present invention a formula (I) aminonitrile may be conveniently prepared by reacting one molar equivalent of a ketone of formula (IV):

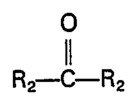

(IV)

wherein $R_1$ and $R_2$ are as hereinabove defined, with a mixture of 3 to 5 molar equivalents of concentrated ammonium hydroxide and of 1.1 to 1.3 molar equivalents of hydrogen cyanide, at a temperature range of from 40 to 45°C, isolating the product thus formed and recycling the mother liquor of the reaction, dissolving in the mother liquor sufficient amounts of ammonia gas and hydrogen cyanide to reestablish the molar equivalent ratios required, as hereinabove defined, and repeating the reaction.

We find, that by the above method, the mother liquor may be recycled at least five times without appreciable losses in yield and purity of the desired formula (I) aminonitrile.

When $R_1$ and $R_2$ of the above formula (I) aminonitrile are different, then the compound of formula (I) is obtained by the above process as the racemic mixture.

Racemic mixtures of the formula (I) aminonitriles may be resolved into their optical isomeric components by customary means, as by selectively precipitating one or the other isomer with the appropriate (+) or (−) optical isomer of an acid, such as tartaric acid. Next, the (+) or (−) aminonitrile is regenerated from its salt with a base such as ammonium hydroxide, isolated and reprecipitated again with the appropriate (+) or (−) acid. Usually, this cycle will have to be repeated several times until the desired optical isomer is obtained in satisfactory optical purity.

The U.S. Patent 3,620,710 discloses the reaction of a ketone, hydrogen cyanide and ammonia. In one of the examples ammonium chloride is dissolved in water, potassium cyanide is added followed by the addition of methylisopropyl ketone after which the reaction mixture is saturated with ammonium gas.

The British patent 1,566,523 describes the preparation of aminonitriles using ammonium chloride and sodium cyanide. However, this patent does not teach or disclose the reaction conditions of this invention.

In order to facilitate a further understanding of the invention, the following examples are presented primarily for the purpose of illustrating certain more specific details thereof.

Example 1

Preparation of 2-amino-2,3-dimethylbutyronitrile

Method

One molar equivalent of methyl isopropyl ketone is added over a 0.5 hour period to a stirred mixture of 3 to 5 molar equivalents of concentrated ammonium hydroxide and 1.20 molar equivalents of hydrogen cyanide. The reaction mixture is stirred at 40—45°C for 2 to 3 hours. The product is extracted from the reaction mixture with methylene chloride and is recovered from the solution by evaporating the solvent.

The aqueous liquor is recycled for the next reaction as follows: 95% by volume of the mother liquor is retained, sufficient amount of anhydrous ammonia is added with cooling to provide the approximately 3 to 5 molar equivalents of ammonia hydroxide per molar equivalent of methyl isopropyl ketone, followed by the addition of 1.02 molar equivalents of hydrogen cyanide per molar equivalent of said ketone.

The reaction is then repeated under the conditions described above. The mother liquor is recycled by the above procedure five times and used in the preparation of the title product. The pertinent data of the six preparations are summarized in Table I below.

TABLE I

Preparation of 2-amino-2,3-dimethylbutyronitrile *via* the recycling of mother liquor on a 0.5 mol scale

| Experiment No. | Recycle No. | 2-amino-2,3-dimethylbutyronitrile % by Wt Yield | % Purity | % Yield |
|---|---|---|---|---|
| 1 | original run | 94.7 | 95.4 | 90.3 |
| 2 | 1st | 96.1 | 95.9 | 92.2 |
| 3 | 2nd | 95.9 | 95.0 | 91.1 |
| 4 | 3rd | 94.7 | 95.9 | 90.8 |
| 5 | 4th | 96.6 | 94.0 | 90.8 |
| 6 | 5th | 96.4 | 94.3 | 90.9 |

It can be clearly seen from the above tabulation that the recycling of the mother liquor has no effect on the overall yields of the reaction and the purity of the product; while the total volume of the mother liquor has been reduced by approximately 80%.

Example 2

Preparation of 2-amino-2,3-dimethylbutyronitrile

By the method of Example 1, the preparation of the title product is repeated on a 9.5 molar scale (a 19x scale-up). There is obtained 988 g of product, 96.7% pure, corresponding to a 89.6% yield.

Example 3

Resolution-racemization of (±)-2-amino-2,3-dimethylbutyronitrile

To a stirred solution of 33.8 g (0.225 mol) of D-(−)-tartaric acid in 120 mL of anhydrous methanol is added 28.1 g (0.25 mol) of (±)-2-amino-2,3-dimethylbutyronitrile. The reaction mixture is stirred at ambient temperature for 22 hours, then the resulting solid is filtered off, washed with 15 mL of methanol, and dried to give 55.4 g of (−)-2-amino-2,3-dimethylbutyronitrile-(−)-hermitartrate. A slurry of 10.4 g of the above salt in 40 mL of water is made basic with 6.8 mL of concentrated ammonia. The solution is extracted with methylene chloride. The organic phase is dried over sodium sulfate and evaporated to afford 3.98 g of (−)-2-amino-2,3-dimethylbutyronitrile $[\alpha]_D^{25} = -6.36°$ (C = 0.00355 g/mL THF).

**Claims**

1. A process for the preparation of a compound of the following formula:

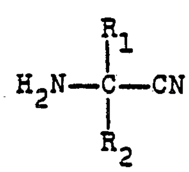

wherein $R_1$ is $C_1$—$C_4$ alkyl; $R_2$ is $C_1$—$C_4$ alkyl or $C_3$—$C_6$ cycloalkyl, and when $R_1$ and $R_2$ are not the same, the racemic mixtures and the optical isomers thereof; characterized by reacting one molar equivalent of a ketone of formula: $R_1CO$—$R_2$, wherein $R_1$ and $R_2$ are as defined above, with a mixture of 3 to 5 molar equivalents of concentrated ammonium hydroxide and of 1.1 to 1.3 molar equivalents of hydrogen cyanide at a temperature range of 40°C to 45°C isolating the product thus formed and recycling the mother liquor of the reaction, dissolving in the mother liquor sufficient amounts of ammonia gas and hydrogen cyanide to re-establish the molar equivalent ratios of 3 to 5 molar equivalents of concentrated ammonium hydroxide and 1.1 to 1.3 molar equivalents of hydrogen cyanide and repeating the reaction.

2. A process according to claim 1 wherein the compound is 2-amino-2,3-dimethylbutyronitrile.

**Patentansprüche**

1. Verfahren zur Herstellung einer Verbindung der folgenden Formel

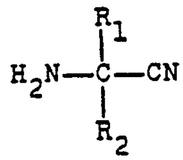

wobei $R_1$ für $C_1$—$C_4$-Alkyl steht; $R_2$ für $C_1$—$C_4$-Alkyl oder $C_3$—$C_6$-Cycloalkyl steht sowie die racemischen Gemische und die optischen Isomere derselben, falls $R_1$ und $R_2$ nicht die gleiche Bedeutung haben; dadurch gekennzeichnet, daß man ein molares Äquivalent eines Ketons der Formel $R_1CO$—$R_2$, wobei $R_1$ und $R_2$ wie oben definiert sind, mit einer Mischung von 3 bis 5 molaren Äquivalenten konzentriertem Ammoniumhydroxid und von 1,1 bis 1,3 molaren Äquivalenten Cyanwasserstoff bei einer Temperatur im Bereich von 40°C bis 45°C umsetzt, das dabei gebildete Produkt isoliert und die Mutterlauge de Umsetzung zurückführt, in der Mutterlauge ausreichende Mengen an Ammoniakgas und Cyanwasserstoff auflöst, um die molaren Äquivalentverhältnisse von 3 bis 5 molaren Äquivalenten konzentriertem Ammoniumhydroxid und 1,1 bis 1,3 molaren Äquivalenten Cyanwassertoff wiederherzustellen, und die Umsetzung wiederholt.

2. Verfahren gemäß Anspruch 1, wobei die Verbindung 2-Amino-2,3-dimethylbutyronitril ist.

**Revendications**

1. Procédé de préparation d'un composé ayant la formula suivante:

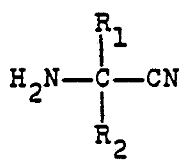

dans laquelle $R_1$ représente un groupe alkyle en $C_1$—$C_4$; $R_2$ représente un groupe alkyle en $C_1$—$C_4$ ou cycloalkyle en $C_3$—$C_6$, et lorsque $R_1$ et $R_2$ ne sont pas identiques, les mélanges racémiques ainsi que les isomères optiques de ceux-ci, caractérisé en ce qu'on fait réagir un équivalent en mole d'une cétone de formule $R_1CO$—$R_2$ dans laquelle $R_1$ et $R_2$ sont tels que défini ci-dessus avec un mélange de 3 à 5 équivalents en mole d'hydroxyde d'ammonium concentré et de 1,1 à 1,3 équivalent en mole de cyanure d'hydrogène à une température de 40°C à 45°C on isole le produit ainsi formé et on recycle les liqueurs mères de la réaction, on dissout dans la liqueur mére, des quantités suffisantes d'ammoniac gazeux et de cyanures d'hydrogène pour rétablir les rapports en équivalent en mole de 3 à 5 équivalents en mole d'hydroxyde d'ammonium concentré et de 1,1 à 1,3 équivalent en mole de cyanure d'hydrogène, et on répète la réaction.

2. Procédé suivant la revendication 1, dans lequel le composé est du 2-amino-2,3-diméthylbutyronitrile.